# FASCICULE DE BREVET EUROPEEN

(11) **EP 4 151 494 B1**
(45) Date de publication et mention de la délivrance du brevet: **12.02.2025**
(21) Numéro de dépôt: 22196357.2
(22) Date de dépôt: 19.09.2022
(51) Int. Cl.: B61D 29/00, A61L 2/10

(54) **VÉHICULE FERROVIAIRE COMPRENANT UN SYSTÈME DE DÉSINFECTION**
SCHIENENFAHRZEUG MIT EINEM DESINFEKTIONSSYSTEM
RAILWAY VEHICLE COMPRISING A DISINFECTION SYSTEM

(30) Priorité: 20.09.2021 FR 2109854
(43) Date de publication de la demande: 22.03.2023
(73) Titulaire: ALSTOM Holdings, 93400 Saint-Ouen-sur-Seine (FR)
(72) Inventeur: GRILLET, Augustin, 59163 Condé sur L'Escaut (FR)
(74) Mandataire: Lavoix

(56) Documents cités:
- EP-A1- 3 378 498
- EP-A1- 3 929 084
- ES-U- 1 255 324
- FR-A1- 3 032 121
- US-A1- 2021 260 229

## Description

La présente invention concerne un véhicule de transport public comprenant un système de désinfection, le système de désinfection comprenant un éclairage à longueur d'onde désinfectante (i.e. à propriété biocide) embarqué à bord dudit véhicule, l'éclairage étant configuré pour désinfecter automatiquement l'intérieur dudit véhicule.

Des systèmes de désinfection par éclairage à longueur d'onde désinfectante sont connus des documents US 2021/260229 A1, FR 3 032 121 A1 et EP 3 929 084 A1.

La présente invention concerne le domaine de la désinfection régulière d'espaces accessibles au public et confrontés à un besoin fréquent, voire régulier, de désinfection, et plus particulièrement la désinfection de l'intérieur de matériel roulant RS (de l'anglais *Rolling Stock*) de transport en commun, notamment au sein d'un véhicule ferroviaire, soit en raison d'un grand nombre de personnes entrant/sortant d'une zone intérieure du véhicule ferroviaire, soit pour des zones plus petites confrontées à une utilisation prolongée par des individus, telles que les toilettes ou la cabine du conducteur du véhicule ferroviaire).

En effet, dans un contexte de pandémie tel que celui relatif au virus Sars-CoV-2 associé à la maladie Covid-19, ou encore pour lutter contre des épisodes de grippes, de gastroentérite, la prolifération de bactéries, etc., une désinfection fréquente de l'intérieur du matériel roulant est nécessaire pour éviter la contamination croisée des passagers touchant des surfaces potentiellement contaminées.

De par leur fonctionnement, de tels matériels roulants, en particulier les plates-formes urbaines et suburbaines pour lesquelles le besoin est le plus élevé, sont fréquemment vides de passagers, notamment sur une plage horaire nocturne, ce qui permet d'effectuer des cycles de décontamination fréquents et sécurisés en l'absence de présence humaine à bord, par exemple lorsque le véhicule ferroviaire a atteint un dépôt ou entre deux services en fin de ligne.

Une première solution pour mettre en oeuvre une telle désinfection (i.e. décontamination) est de la réaliser principalement par des opérations manuelles de désinfection chimique. Cependant une telle première solution est très coûteuse, longue, sujette aux erreurs humaines et peu respectueuse de l'environnement.

Pour y remédier, des solutions de désinfection de matériel roulant de transport en commun ont été développées et sont par exemple basées sur l'usage de lampes à rayonnement ultraviolet UV-C par ailleurs déjà utilisées dans les secteurs de la santé et de l'agroalimentaire.

Plus précisément, pour mettre en oeuvre ces solutions de désinfection de matériel roulant de transport en commun basées sur un éclairage UV-C, des opérateurs installent chaque nuit un tel éclairage UV-C pendant le service de maintenance dans chaque voiture de véhicule ferroviaire. Cependant, de telles lampes UV-C sont généralement placées de manière assez arbitraire dans l'habitacle du véhicule, et ne permettent pas de délivrer une dose homogène d'énergie UV-C sur toutes les surfaces à désinfecter.

La désinfection est une question de dosage de l'énergie rayonnée par l'éclairage, un tel dosage correspondant à une combinaison de puissance d'éclairage et de temps d'éclairage.

Les longueurs d'onde s'atténuent généralement en fonction de la distance parcourue, ce qui nécessite d'augmenter la puissance de telles lampes pour assurer un rayonnement suffisant sur les surfaces les plus éloignées.

De plus, lorsque l'on est limité par des aspects opérationnels tels que le temps, le nombre de véhicules/voitures ferroviaires à désinfecter, le nombre de lampes disponibles, il n'y a généralement pas d'autre choix que d'utiliser des lampes très puissantes pour pouvoir traiter plus de zones à désinfecter par unité de temps.

Toutefois, une telle augmentation de puissance s'avère coûteuse et entraîne en contrepartie des doses de rayonnement élevées pour les surfaces proches de la lampe, et une dégradation (i.e. vieillissement) accélérée résultante de ces surfaces à proximité.

En outre, en raison du danger potentiel de la lumière UV-C pour les humains, notamment pour les yeux et la peau, son utilisation pour la désinfection quotidienne du matériel roulant reste complexe et coûteuse.

L'un des buts de l'invention est de pallier les inconvénients précités en proposant une solution de désinfection d'un véhicule ferroviaire à la fois efficace pour traiter l'ensemble des zones de fort contact passager, autonome et sécurisée, et facilement intégrable.

A cet effet, l'invention concerne un véhicule de transport public, notamment un véhicule ferroviaire comprenant un système de désinfection, le système de désinfection comprenant un éclairage à longueur d'onde désinfectante embarqué à bord dudit véhicule, l'éclairage comprenant au moins un dispositif d'éclairage à longueur d'onde désinfectante, l'éclairage étant configuré pour désinfecter automatiquement l'intérieur dudit véhicule, ledit éclairage étant automatiquement contrôlé de manière centralisée par un système de contrôle et de surveillance dudit véhicule.

Selon un mode de réalisation particulier, le véhicule comprend une ou plusieurs des caractéristiques suivantes, prises isolément ou selon toutes les combinaisons techniquement possibles :
- l'éclairage à longueur d'onde désinfectante comprend une pluralité de dispositifs d'éclairage à longueur d'onde désinfectante répartis au sein dudit véhicule, parallèlement au plafond dudit véhicule, dans au moins un des espaces suivants dudit véhicule :
   - un compartiment passager,
   - un espace toilettes,
   - une cabine conducteur,
   - un espace de transport marchandise,
   - un espace de restauration,
   - une plateforme d'entrée/sortie passager à bord dudit véhicule ;
- le système de contrôle et de surveillance dudit véhicule est configuré pour activer chaque dispositif d'éclairage selon une fréquence prédéterminée associée au type d'espace dans lequel ledit dispositif d'éclairage est installé ;
- chaque dispositif d'éclairage est un éclairage à rayonnement ultra-violet UV-C appartenant au groupe comprenant au moins :
   - une lampe UV-C à tube, pulsée ou à onde continue ;
   - une lampe UV-C à laser ;
   - au moins une diode électroluminescente UV-C connectée(s) au système de contrôle et de surveillance dudit véhicule via un convertisseur statique ;
- un dispositif d'éclairage correspondant à au moins une diode électroluminescente UV-C connectée(s) au système de contrôle et de surveillance dudit véhicule via un convertisseur statique est propre à être combiné avec au moins une diode électroluminescente configurée pour fournir une lumière blanche ;
- chaque dispositif d'éclairage est propre à être intégré au sein d'un équipement d'éclairage comprenant un verre ou un polymère transparent de diffusion du rayonnement produit par ledit dispositif d'éclairage vers l'intérieur dudit véhicule ;
- l'activation d'au moins un desdits dispositifs d'éclairage est asservie à l'absence de présence humaine détectée par au moins un détecteur de présence installé à proximité dudit dispositif d'éclairage ;
- le véhicule comprend en outre au moins une surface configurée pour réfléchir l'éclairage à longueur d'onde désinfectante dudit système de désinfection ;
- le véhicule comprend en outre une boucle de rétroaction, connectée audit système de contrôle et de surveillance dudit véhicule, et configurée pour optimiser l'activation dudit éclairage à longueur d'onde désinfectante au moyen d'un capteur appartenant au groupe comprenant au moins :
   - un capteur actif de dosimétrie propre à quantifier l'énergie reçue associée au rayonnement dudit éclairage à longueur d'onde désinfectante, ledit capteur actif étant installé dans un emplacement prédéterminé au sein dudit véhicule, dans lequel ledit capteur est propre à recevoir ledit rayonnement dudit éclairage à longueur d'onde désinfectante;
   - un capteur actif de dosimétrie propre à quantifier l'énergie reçue associée audit rayonnement dudit éclairage à longueur d'onde désinfectante, ledit capteur actif étant installé au niveau de l'éclairage à longueur d'onde désinfectante et combiné à une surface réflectrice dont l'emplacement au sein dudit véhicule est configuré pour la faire réfléchir le rayonnement reçu en direction dudit capteur actif installé au niveau de l'éclairage à longueur d'onde désinfectante ;
   - un capteur passif propre à réagir physiquement au rayonnement dudit éclairage à longueur d'onde désinfectante, la réaction physique dudit capteur passif étant représentative d'un rayonnement dudit éclairage à longueur d'onde désinfectante supérieur à un seuil prédéterminé ;
- au moins un élément dudit véhicule propre à être illuminé par ledit éclairage à longueur d'onde désinfectante est recouvert d'un revêtement configuré pour le protéger d'un vieillissement accéléré par ledit rayonnement dudit éclairage à longueur d'onde désinfectante.

D'autres caractéristiques et avantages de l'invention ressortiront de la description détaillée qui en est donnée ci-dessous, à titre indicatif et nullement limitatif, en référence aux figures annexées, parmi lesquelles :
[Fig 1] la figure 1 illustre schématiquement un véhicule, notamment ferroviaire, comprenant un système de désinfection selon un premier mode de réalisation de l'invention ;
[Fig 2] la figure 2 illustre l'intégration d'un système de désinfection au sein d'un véhicule, notamment ferroviaire, selon une variante particulière de la présente invention.

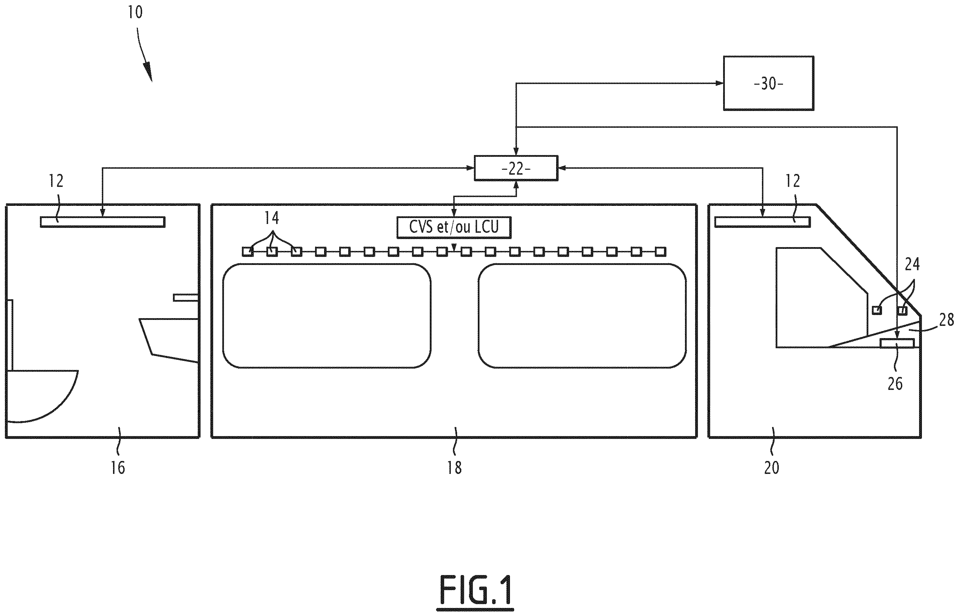

La figure 1 représente un véhicule 10 de transport public selon un premier mode de réalisation de l'invention, un tel véhicule 10 comprenant un système de désinfection. Le véhicule 10 est ici un véhicule ferroviaire. En variante, le véhicule 10 est un véhicule routier, tel un bus ou un car, un véhicule aérien ou un véhicule naval.

Le système de désinfection comprend un éclairage à longueur d'onde désinfectante (i.e. à propriété biocide) embarqué à bord dudit véhicule ferroviaire 10, l'éclairage comprenant au moins un dispositif d'éclairage à longueur d'onde désinfectante l'éclairage étant configuré pour désinfecter automatiquement l'intérieur dudit véhicule ferroviaire.

Selon un aspect particulier non représenté, il est notamment à noter que des éclairages à longueurs d'onde visibles, notamment comprises entre 400 et 420nm, sont propres à avoir un effet désinfectant, ce qui est propre à permettre une intégration au sein d'un éclairage existant par exemple à lumière blanche.

Selon un exemple particulier, le système de désinfection selon la présente invention est basé sur un éclairage ultraviolet UV-C associé à une bande spectrale de 100 à 280 nm.

En particulier, chaque dispositif d'éclairage propre à fournir un éclairage à rayonnement ultra-violet UV-C appartient au groupe comprenant au moins : une lampe UV-C à tube (gaz), pulsée ou à onde continue, une lampe UV-C à laser, au moins une diode électroluminescente UV-C, chaque éclairage à rayonnement ultra-violet UV-C dudit groupe étant propre à être connecté au système de contrôle et de surveillance 22 dudit véhicule ferroviaire via un convertisseur statique CVS (de l'anglais *Continuously Variable Slope Delta modulation*), et/ou via un module, d'adaptation du faisceau de lumière LCU (de l'anglais *Light Control Unit*), un tel module LCU étant propre à introduire une modulation de largeur d'impulsions PWM (de l'anglais *Pulse Width Modulation*) pour par exemple contrôler l'intensité des LED.

Selon l'exemple particulier de la figure 1, un tel éclairage ultraviolet UV-C comprend l'utilisation de lasers ou de tubes 12 connectés à un convertisseur statique CVS et/ou à un LCU, non représenté sur la figure 1, propre à fournir un courant en basse tension, plus encombrants, et/ou l'utilisation de spots lumineux 14 à diodes électroluminescentes UV-C, également appelées LED UV-C (de l'anglais *light-emitting diode*) connectées à un convertisseur statique CVS et/ou à un LCU, de telles LED UV-C étant désormais technologiquement disponibles et dont le niveau de puissance atteignable ne cesse d'augmenter avec le progrès technologique, ce qui permet leur intégration compacte dans les intérieurs de matériel roulant ferroviaire, voire même au sein de luminaires d'éclairage général existants au sein du véhicule ferroviaire. En effet, les LED UV-C sont propres à être facilement couplées à des microcontrôleurs sur des circuits imprimés, ce qui permet de concevoir et d'intégrer un système autonome intelligent dans les plafonds ou les arches de toit du véhicule ferroviaire tel qu'illustré par la figure 1.

En particulier, un ensemble de diodes électroluminescente UV-C permet de mieux distribuer au sein du volume à désinfecter l'illumination et d'éviter des disparités d'illumination avec des zones fortement illuminées et possiblement dégradées et des zones trop faiblement illuminées pour être désinfectées.

Tel qu'illustré sur l'exemple de la figure 1, le véhicule ferroviaire selon la présente invention est propre à présenter une combinaison de ces deux types d'éclairage ultraviolet UV-C, en installant par exemple des éclairages ultraviolet UV-C à lasers ou à tubes 12 connectés à un convertisseur statique CVS, non représenté, au sein d'un espace toilettes 16, des spots 14 à diodes électroluminescentes UV-C connecté(e)s à un convertisseur statique CVS dans le plafond d'une voiture passager 18 du véhicule ferroviaire 10, et des éclairages ultraviolet UV-C à lasers ou à tubes 12 connectés à un convertisseur statique CVS, non représenté, au sein d'une cabine conducteur 20. La simulation d'éclairage en amont permet de concevoir un schéma d'éclairage distribué optimal adapté à la conception même du volume à traiter. Un tel schéma d'éclairage distribué optimal permet d'utiliser des éclairages moins puissants dans certaines zones, notamment au moyen des spots 14 à diodes électroluminescentes UV-C, et ainsi d'utiliser des conditions d'éclairage moins dégradantes (i.e. détériorantes) pour les surfaces à proximité des lampes 12.

Autrement dit, l'éclairage à longueur d'onde désinfectante (i.e. à propriété biocide) comprend une pluralité de dispositifs d'éclairage à longueur d'onde désinfectante répartis au sein dudit véhicule ferroviaire 10, parallèlement au plafond dudit véhicule, dans au moins un des espaces suivants dudit véhicule ferroviaire, un compartiment passager 18, un espace toilettes 16, une cabine conducteur 20, ou encore de manière non représentée un espace de transport marchandise, un espace de restauration, une plateforme d'entrée/sortie passager à bord dudit véhicule ferroviaire, etc.

Avantageusement et spécifiquement selon la présente invention, chaque élément du système d'éclairage à longueur d'onde désinfectante (i.e. à propriété biocide) embarqué à bord dudit véhicule ferroviaire 10 est automatiquement contrôlé de manière centralisée par un système 22 de contrôle et de surveillance TCMS (de l'anglais Train Control and Monitoring System) dudit véhicule ferroviaire 10.

Un tel contrôle centralisé par TCMS 22 proposé spécifiquement selon la présente invention permet notamment de programmer facilement l'activation/désactivation de cycles d'illumination, le cas échéant, comme détaillé par la suite, en couplage avec un ou plusieurs détecteur(s) de présence au sein du véhicule ferroviaire et/ou avec tout autre mécanisme de sécurité du système ferroviaire du véhicule ferroviaire 10 considéré, afin de permettre un contrôle automatisé à distance en évitant la présence humaine d'opérateurs sur site.

Tel qu'illustré sur la figure 1, l'utilisation de spots lumineux 24 à diodes électroluminescentes UV-C est propre à également être mise en oeuvre au sein de la cabine conducteur 20, et le TCMS 22 est propre à contrôler un bouton d'activation/désactivation 26 localisé au sein du tableau de bord 28 de cette cabine conducteur 20 ou dans un cabinet électrique protégé situé à l'extérieur de la cabine. Les spots lumineux 24 à diodes électroluminescentes UV-C permettent notamment de traiter le tableau de bord 28, et sont reliés au TCMS 22 (bien que cela ne soit pas explicitement représenté sur la figure 1).

Selon la présente invention, le TCMS 22 est propre à générer un ensemble 30 comprenant au moins une programmation, et/ou des instructions de contrôle du système d'éclairage à longueur d'onde désinfectante (i.e. à propriété biocide), et/ou un rapport de fonctionnement associé.

Selon un aspect particulier, le système de contrôle et de surveillance 22 du véhicule ferroviaire 10 est configuré pour activer chaque dispositif d'éclairage selon une fréquence prédéterminée associée au type d'espace dans lequel ledit dispositif est installé.

Les sources lumineuses désinfectantes (i.e. à propriété biocide), notamment UV-C, intégrées à l'intérieur du matériel roulant à des endroits stratégiques et avec une puissance optimale, notamment en fonction de simulations d'éclairage et d'autres dispositions spécifiques peuvent être activées à distance pour effectuer une désinfection régulière et automatique de l'air et des surfaces internes du véhicule contre les agents pathogènes, y compris les virus tels que le SARS-COV2.

Un exemple de programmation visant par exemple à programmer : l'activation des éclairages à longueur d'onde désinfectante, notamment à ultraviolet UV-C, à lasers ou à tubes 12 dans l'espace toilettes 16 entre chaque passage de passager, l'activation des spots 14 à diodes électroluminescentes UV-C connecté(e)s à un convertisseur statique CVS et/ou à un LCU dans le plafond d'une voiture passager 18 du véhicule ferroviaire 10 entre deux services distincts, et l'activation de l'éclairage à longueur d'onde désinfectante (i.e. à propriété biocide), notamment à ultraviolet UV-C, à lasers ou à tubes 12 ou à LED 24 au sein de la cabine conducteur 20 entre chaque changement de conducteur.

En termes de dosage, correspondant à une combinaison de puissance d'éclairage et de temps d'éclairage, un exemple de dose significative, notamment en termes d'effet antimicrobien y compris sur les virus tels que le SARS-COV2, est de l'ordre de 10mJ/cm² ou 10W/m² dans le cas d'un éclairage de 10 secondes. Un tel dosage dépend du spectre d'émission exact de l'éclairage à longueur d'onde désinfectante utilisé, ainsi que de la nature de la surface à traiter, notamment en termes de type de matériau, de finition de surface, état de propreté, etc. et doit donc être affiné en conséquence.

A titre d'exemple, un éclairage UV-C d'une heure, comme cela pourrait être le cas entre deux services pour décontaminer des voitures passager 18 nécessite par exemple une puissance de l'ordre de 0,028W/m², un éclairage de cinq minutes, comme cela pourrait être le cas entre deux changements de conducteur, nécessite par exemple une puissance de l'ordre de 0,33W/m², un éclairage de dix secondes, par exemple pour traiter les toilettes entre deux occupations, nécessite par exemple une puissance de l'ordre de 10W/m².

Le contrôle automatique et centralisé mis en oeuvre selon la présente invention offre une flexibilité de traitement, propre à être initiée sur demande ou de manière diurne au lieu d'avoir à attendre la durée d'un service complet se terminant le plus souvent en fin de journée. En conséquence, les intérieurs de matériel roulant peuvent être maintenus sanitairement sécurisés tout au long de la journée, au lieu d'être potentiellement de plus en plus contaminés au fur et à mesure du service, notamment au niveau de la cabine conducteur afin de permettre une décontamination entre changement de conducteur, sans qu'un retour au dépôt ou l'envoi d'une équipe de décontamination manuelle ne soit nécessaire.

Par ailleurs, il est à noter que les spots 14 à diodes électroluminescentes UV-C présentent l'avantage d'être facilement intégrables au sein d'un intérieur roulant, car de taille et de puissance réduite par rapport à l'éclairage classique à base de lasers ou de tubes 12, ce qui permet de notamment de les intégrer sous forme de spots à diodes électroluminescentes UV-C.

De tels spots à diodes électroluminescentes UV-C rapprochés de la surface peuvent fournir une dose d'UV-C similaire, mais par le biais d'une illumination plus douce, par rapport à une lampe pulsée ou non pulsée 12, ce qui sera moins dommageable pour la surface intérieure, dans les zones plus critiques, notamment là où il y a beaucoup de plastiques. Ceci est particulièrement avantageux en tenant compte du fait que l'intensité du rayonnement lumineux s'atténue fortement sur la distance de déplacement, notamment de manière proportionnelle au carré de la distance. Ainsi, les spots 14 à diodes électroluminescentes UV-C, propres à être intégrés plus proches des surfaces à traiter, ont clairement un avantage en termes d'efficacité globale.

Selon une première option d'intégration, un dispositif d'éclairage correspondant à au moins une diode électroluminescente UV-C, ou correspondant à un éclairage à longueur d'onde désinfectante (i.e. à propriété biocide), connectée(s) au système de contrôle et de surveillance 22 dudit véhicule ferroviaire 10 via un convertisseur statique CVS est propre à être combiné avec au moins une diode électroluminescente configurée pour fournir une lumière blanche d'éclairage général ou d'éclairage d'ambiance. Une telle combinaison aurait une double fonction offrant ainsi une solution très efficace en termes d'intégration et d'espace, les diodes électroluminescente UV-C, et les diodes électroluminescentes étant propres à être activées séparément. Une telle combinaison est propre à diffuser de la lumière à travers une surface transparente pour une gamme prédéterminée de longueurs d'ondes visibles et UV-C, correspondant notamment à un verre ou à un polymère transparent de diffusion du rayonnement produit par ledit dispositif d'éclairage vers l'intérieur du véhicule ferroviaire 10, une telle surface transparente pour une gamme prédéterminée de longueurs d'ondes visibles laissant également passer notamment le rayonnement UV-C lors de son activation.

Selon une deuxième option, des dispositifs dédiés uniquement à la fourniture d'un éclairage UV-C sont mis en oeuvre, notamment selon une première variante, sous la forme de rampes à diodes électroluminescentes UV-C, comprenant par exemple deux lignes superposée de six LED UV-C, le nombre six étant donné à titre d'exemple tout en restant totalement arbitraire, selon une deuxième variante sous la forme de deux lampes à gaz UV-C (i.e. un tube) superposées au lieu des deux lignes superposée de six LED UV-C, ou encore selon une troisième variante sous la forme de spots individuels, chaque spot comprenant une seule LED UV-C ces trois variantes étant propres à diffuser de la lumière à travers une surface transparente pour une gamme prédéterminée de longueurs d'ondes visibles et UV-C, correspondant notamment à un verre ou à un polymère transparent de diffusion du rayonnement produit par ledit dispositif d'éclairage vers l'intérieur du véhicule ferroviaire 10.

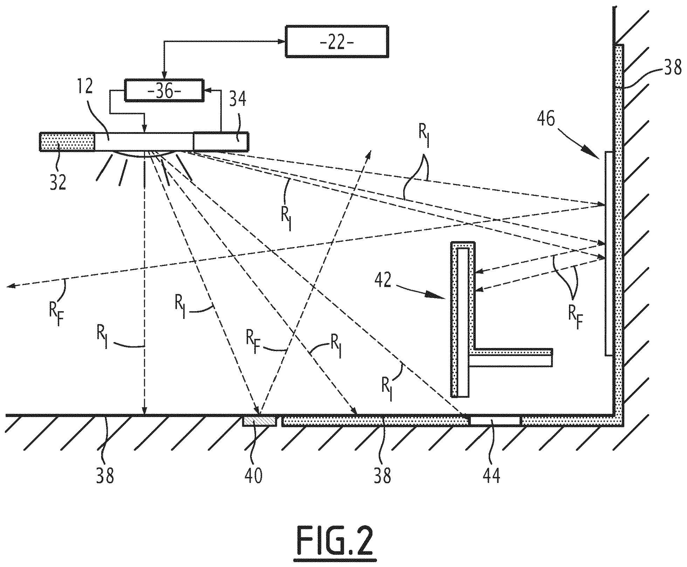

En complément facultatif, tel qu'illustré par la figure 2, l'activation d'au moins un desdits dispositifs d'éclairage à longueur d'onde désinfectante, notamment une lampe UV-C 12, est asservie à l'absence de présence humaine au sein du véhicule ferroviaire 10, l'absence de présence humaine étant détectée par au moins un détecteur de présence 32 installé à proximité dudit dispositif d'éclairage 12 et/ou par analyse des flux vidéos des caméras de vidéosurveillance intégrées au matériel roulant.

Un tel aspect vient notamment compléter des mécanismes de sécurité intégrés, habituellement présents dans les véhicules ferroviaires, et garantissant que les cycles d'éclairage UV-C ne peuvent être activés que lorsque personne ne se trouve à bord. Autrement dit, selon cet aspect complémentaire facultatif, les dispositifs d'éclairage à longueur d'onde désinfectante, en particulier les éclairages de type UV-C sont propres à être couplées à des capteurs de détection de présence 32 intégrés, de sorte que la lumière s'éteigne automatiquement dès qu'un être humain est détecté dans le champ d'éclairage.

En complément facultatif, selon le mode de réalisation particulier illustré par la figure 2, le véhicule ferroviaire 10 comprend en outre une boucle de rétroaction, connectée audit système 22 de contrôle et de surveillance dudit véhicule ferroviaire, et configurée pour optimiser l'activation automatique dudit éclairage 12 à longueur d'onde désinfectante au moyen d'un capteur correspondant à un capteur actif de dosimétrie 34 propre à quantifier l'énergie reçue associée audit rayonnement dudit éclairage à longueur d'onde désinfectante.

Comme illustré par la figure 2, un tel dosimètre 34 rétroagit, via un microcontrôleur 36 et/ou le système de contrôle TCMS 22 sur l'activation de la lampe 12.

Le capteur actif de dosimétrie 34 est notamment installé au niveau de (i.e. dans un plan sensiblement égal à celui de) l'éclairage 12 à longueur d'onde désinfectante et combiné, par exemple au niveau du sol 38, à une surface réflectrice 40 dont l'emplacement au sein dudit véhicule ferroviaire (pas nécessairement au sol) est configuré pour la faire réfléchir le rayonnement reçu en direction dudit capteur actif de dosimétrie 34 installé au niveau de l'éclairage à longueur d'onde désinfectante.

Autrement dit, selon le mode de réalisation de la figure 2, le capteur actif de dosimétrie 34 est protégé de l'illumination directe du composant émetteur, tel que la lampe UV-C 12, et un composant réfléchissant 40 les UV-C (i.e. réflecteur UV-C) est placé avantageusement sur le sol 38 de la surface intérieure du matériel roulant pour agir comme un réflecteur calibré. Un tel mode de réalisation présente l'avantage de permettre une intégration facilitée en utilisant une carte électronique unique d'activation de l'éclairage 12 avec une boucle de rétroaction intégrée.

Un réflecteur UV-C 40 correspond par exemple à un morceau de métal tel que l'aluminium ou le PTFE ou e-PTFE qui présente une réflectivité UV-C proche de 95 %.

La mise en œuvre d'une telle boucle de rétroaction est avantageuse notamment pour assurer un contrôle de la qualité par la conception, pour compenser les effets du vieillissement des lampes, les lampes UV-C ayant tendance à vieillir assez rapidement, et pour pouvoir éteindre l'éclairage dès que la dose nécessaire a été délivrée aux surfaces afin de ne pas dégrader inutilement les matériaux.

A titre d'alternative au mode de réalisation de la figure 2, le capteur actif de dosimétrie propre à quantifier l'énergie reçue associée au rayonnement dudit éclairage à longueur d'onde désinfectante, au lieu d'être installé au niveau de (i.e. dans un plan sensiblement égal à celui de) l'éclairage 12 à longueur d'onde désinfectante, est installé dans un emplacement prédéterminé au sein dudit véhicule ferroviaire 10, dans lequel ledit capteur est propre à recevoir directement ledit rayonnement dudit éclairage à longueur d'onde désinfectante, par exemple à la place du réflecteur 40 au niveau du sol 38 représenté sur la figure 2.

L'emplacement prédéterminé d'installation du capteur actif de dosimétrie est notamment propre à faciliter sa connexion au système 22 de contrôle et de surveillance TCMS, une telle connexion étant propre à être directe ou via le microcontrôleur 36 représenté sur la figure 2, ou l'emplacement prédéterminé est propre à faciliter sa propre maintenance, etc., et a été étalonné au préalable.

Un tel capteur actif de dosimétrie correspond notamment à une photodiode sensible aux UV-C.

Selon une autre alternative, le capteur actif de dosimétrie est remplacé par un capteur passif (i.e. dosimètre passif) propre à réagir physiquement au rayonnement dudit éclairage à longueur d'onde désinfectante, la réaction physique dudit capteur passif étant représentative d'un rayonnement dudit éclairage à longueur d'onde désinfectante supérieur à un seuil prédéterminé, un tel seuil prédéterminé correspondant à l'atteinte de la dose d'illumination nécessaire pour obtenir un niveau de décontamination prédéterminé.

En particulier, la réaction physique d'un tel capteur passif intégré au sein de la boucle de rétroaction correspond à un changement de couleur, et l'utilisation d'un tel capteur passif au sein de la boucle de rétroaction présente l'avantage d'être économique en comparaison avec les variantes de boucle de rétroaction intégrant un capteur actif.

Le contrôle de qualité de la réaction du capteur passif est, selon une première variante propre à être effectué par un opérateur, ou selon une deuxième variante, si le dosimètre est placé avantageusement dans le champ de vision d'une caméra embarquée à bord du véhicule ferroviaire, notamment une caméra de système de surveillance et de contrôle CCTV (de l'anglais *Closed-Circuit TeleVision*) existante au sein de l'habitacle du matériel roulant du véhicule ferroviaire, propre à être effectué au moyen d'une telle caméra, en utilisant notamment un algorithme d'analyse d'image et de détection de couleur, pour émettre un signal de contrôle vers le système de contrôle et de surveillance TCMS 22.

Selon un autre complément facultatif, afin de réduire le nombre de dispositifs d'éclairage à longueur d'onde désinfectante répartis au sein dudit véhicule ferroviaire et nécessaires pour couvrir exhaustivement la désinfection de l'ensemble de la surface intérieure et traiter, du fait de la présence d'obstacle 42 au rayonnement incident R_{I}, des zones d'ombres 44, tel qu'illustré en présence d'un siège au sein de la figure 2, le véhicule ferroviaire 10 comprend en outre au moins une surface 46 configurée pour réfléchir, tel qu'illustré par les rayons R_{F} sur la figure 2, l'éclairage à longueur d'onde désinfectante dudit système de désinfection.

En particulier, grâce à la surface réfléchissante 46, la taille de la surface 44 est réduite et inférieure à l'assise du siège 42, alors qu'en l'absence d'une telle surface réfléchissante 46 la taille de la zone d'ombre 44, non atteinte par l'éclairage à longueur d'onde désinfectante et donc non décontaminé serait supérieure à celle de l'assise du siège 42.

Par exemple, une telle surface réfléchissante 46 correspond à un matériau, revêtement ou à une finition de surface, notamment sous la forme d'un film transparent présentant une réflectivité UV-C optimisée, de sorte qu'au moins une partie des zones qui ne se trouvent pas dans la ligne de visée directe du luminaire UV-C puissent tout de même être éclairées.

Par exemple, des revêtements réfléchissant les rayons UV-C ont notamment été développés pour des applications spatiales, où le rayonnement UV-C est propre à se présenter.

En complément facultatif, non représenté, au moins un élément dudit véhicule ferroviaire, propre à être illuminé par ledit éclairage à longueur d'onde désinfectante, est recouvert d'un revêtement configuré pour le protéger d'un vieillissement accéléré par ledit rayonnement dudit éclairage à longueur d'onde désinfectante.

Une tel aspect complémentaire facultatif permet notamment d'offrir une protection des matériaux de matériel roulant contre le vieillissement induit par les rayonnements à longueur d'onde désinfectante, notamment UV-C. En effet, certains composants de matériel roulant sont propres à utiliser des matériaux très sensibles au rayonnement UV-C. Au lieu de chercher un matériau alternatif, un tel revêtement de protection UV-C, notamment sous forme de film transparent, est propre à être appliqué sur les composants fragiles identifiés, tels que les thermoplastiques, les polycarbonates, les peintures, etc.

L'homme du métier comprendra que l'invention ne se limite pas aux modes de réalisation décrits, ni aux exemples particuliers de la description, les modes de réalisation et les variantes mentionnées ci-dessus étant propres à être combinés entre eux pour générer de nouveaux modes de réalisation de l'invention.

La présente invention propose ainsi une solution désinfection de l'intérieur du matériel roulant ferroviaire par intégration d'un éclairage à longueur d'onde désinfectante avec contrôle à distance facilement réalisable via le TCMS existant d'un véhicule ferroviaire.

Un tel contrôle à distance de l'activation de l'éclairage à longueur d'onde désinfectante, notamment UV-C, permet de mettre en œuvre une désinfection automatique et régulière (i.e. périodique), permettant d'augmenter la sécurité lors des opérations de désinfection, la présence humaine au sein du véhicule ferroviaire étant évitée, et offrant une flexibilité de désinfection avec une augmentation de fréquence de désinfection y compris diurne voire à la demande dès qu'une période d'inutilisation se présente.

Une solution telle solution d'éclairage à effet désinfectant, notamment UV-C intégrée et contrôlable à distance est avantageusement économique, car réduisant des coûts d'investissement et d'exploitation pour un opérateur de véhicule tel qu'un opérateur ferroviaire, et augmentant la durée de vie des intérieurs de matériel roulant ferroviaire par rapport à d'autres solutions d'éclairage UV-C, ce qui se traduit par une fréquence de remise en état (i.e. maintenance) réduite. De plus, d'un point de vue sécurité, la présente invention basée sur un contrôle automatique de désinfection à distance permet de réduire les risques d'accidents potentiels impliquant des opérateurs humains, et de par son caractère visible est propre à rassurer les passagers.

## Revendications

1. Véhicule (10) ferroviaire, comprenant un système de désinfection, le système de désinfection comprenant un éclairage à longueur d'onde désinfectante embarqué à bord dudit véhicule (10), l'éclairage comprenant au moins un dispositif (12, 14) d'éclairage à longueur d'onde désinfectante, l'éclairage étant configuré pour désinfecter automatiquement l'intérieur dudit véhicule, le véhicule étant **caractérisé en ce que** ledit éclairage est automatiquement contrôlé de manière centralisée par un système TCMS de contrôle et de surveillance (22) dudit véhicule (10).

2. Véhicule (10) selon la revendication 1, dans lequel l'éclairage à longueur d'onde désinfectante comprend une pluralité de dispositifs (12, 14) d'éclairage à longueur d'onde désinfectante répartis au sein dudit véhicule, parallèlement au plafond dudit véhicule, dans au moins un des espaces suivants dudit véhicule :
- un compartiment passager (18),
- un espace toilettes (16),
- une cabine conducteur (20),
- un espace de transport marchandise,
- un espace de restauration,
- une plateforme d'entrée/sortie passager à bord dudit véhicule

3. Véhicule (10) selon la revendication 2, dans lequel le système de contrôle et de surveillance (22) dudit véhicule est configuré pour activer chaque dispositif d'éclairage (12, 14) selon une fréquence prédéterminée associée au type d'espace (16, 18, 20) dans lequel ledit dispositif d'éclairage est installé.

4. Véhicule (10) selon l'une quelconque des revendications 1 à 3, dans lequel chaque dispositif d'éclairage est un éclairage à rayonnement ultra-violet UV-C appartenant au groupe comprenant au moins :
- une lampe UV-C à tube (12), pulsée ou à onde continue ;
- une lampe UV-C à laser ;
- au moins une diode électroluminescente UV-C (14) connectée(s) au système de contrôle et de surveillance (22) dudit véhicule via un convertisseur statique.

5. Véhicule (10) selon la revendication 4, dans lequel un dispositif d'éclairage correspondant à au moins une diode électroluminescente UV-C (14) connectée(s) au système (22) de contrôle et de surveillance dudit véhicule via un convertisseur statique est propre à être combiné avec au moins une diode électroluminescente configurée pour fournir une lumière blanche.

6. Véhicule (10) selon la revendication 4 ou 5, dans lequel chaque dispositif d'éclairage est propre à être intégré au sein d'un équipement d'éclairage comprenant un verre ou un polymère transparent de diffusion du rayonnement produit par ledit dispositif d'éclairage vers l'intérieur dudit véhicule.

7. Véhicule (10) selon l'une quelconque des revendications 1 à 6, dans lequel l'activation d'au moins un desdits dispositifs d'éclairage est asservie à l'absence de présence humaine détectée par au moins un détecteur (32) de présence installé à proximité dudit dispositif d'éclairage.

8. Véhicule (10) selon l'une quelconque des revendications précédentes comprenant en outre au moins une surface (40, 46) configurée pour réfléchir l'éclairage à longueur d'onde désinfectante dudit système de désinfection.

9. Véhicule (10) selon l'une quelconque des revendications précédentes, comprenant en outre une boucle de rétroaction, connectée audit système (22) de contrôle et de surveillance dudit véhicule, et configurée pour optimiser l'activation dudit éclairage à longueur d'onde désinfectante au moyen d'un capteur appartenant au groupe comprenant au moins :
- un capteur actif de dosimétrie propre à quantifier l'énergie reçue associée au rayonnement dudit éclairage à longueur d'onde désinfectante, ledit capteur actif étant installé dans un emplacement prédéterminé au sein dudit véhicule, dans lequel ledit capteur est propre à recevoir ledit rayonnement dudit éclairage à longueur d'onde désinfectante ;
- un capteur actif de dosimétrie (34) propre à quantifier l'énergie reçue associée audit rayonnement dudit éclairage à longueur d'onde désinfectante, ledit capteur actif étant installé au niveau de l'éclairage à longueur d'onde désinfectante et combiné à une surface réflectrice dont l'emplacement au sein dudit véhicule ferroviaire est configuré pour la faire réfléchir le rayonnement reçu en direction dudit capteur actif installé au niveau de l'éclairage à longueur d'onde désinfectante ;
- un capteur passif propre à réagir physiquement au rayonnement dudit éclairage à longueur d'onde désinfectante, la réaction physique dudit capteur passif étant représentative d'un rayonnement dudit éclairage à longueur d'onde désinfectante supérieur à un seuil prédéterminé.

10. Véhicule (10) selon l'une quelconque des revendications précédentes, dans lequel au moins un élément dudit véhicule ferroviaire propre à être illuminé par ledit éclairage à longueur d'onde désinfectante est recouvert d'un revêtement configuré pour le protéger d'un vieillissement accéléré par ledit rayonnement dudit éclairage à longueur d'onde désinfectante.

## Patentansprüche

1. Schienenfahrzeug (10), umfassend ein Desinfektionssystem, das Desinfektionssystem umfassend eine Beleuchtung mit desinfizierender Wellenlänge an Bord des Fahrzeugs (10), die Beleuchtung umfassend mindestens eine Beleuchtungsvorrichtung (12, 14) mit desinfizierender Wellenlänge, wobei die Beleuchtung konfiguriert ist, um das Innere des Fahrzeugs automatisch zu desinfizieren, wobei das Fahrzeug **dadurch gekennzeichnet ist, dass** die Beleuchtung automatisch zentral von einem TCMS-Steuerungs- und Überwachungssystem (22) des Fahrzeugs (10) gesteuert wird.

2. Fahrzeug (10) nach Anspruch 1, wobei die Beleuchtung mit desinfizierender Wellenlänge eine Vielzahl von Beleuchtungsvorrichtungen (12, 14) mit desinfizierender Wellenlänge umfasst, die innerhalb des Fahrzeugs parallel an Decke des Fahrzeugs in mindestens einem der folgenden Räume des Fahrzeugs verteilt sind:
- einem Passagierabteil (18),
- einem Toilettenbereich (16),
- einer Fahrerkabine (20),
- ein Warentransportbereich,
- einem Restaurantbereich,
- einer Einstiegs-/Ausstiegsplattform für Passagiere an Bord des Fahrzeugs

3. Fahrzeug (10) nach Anspruch 2, wobei das Steuer- und Überwachungssystem (22) des Fahrzeugs konfiguriert ist, um jede Beleuchtungsvorrichtung (12, 14) mit einer vorbestimmten Frequenz zu aktivieren, die mit der Art des Bereichs (16, 18, 20) assoziiert ist, in dem die Beleuchtungsvorrichtung installiert ist.

4. Fahrzeug (10) nach einem der Ansprüche 1 bis 3, wobei jede Beleuchtungsvorrichtung eine Beleuchtung mit ultravioletter UV-C-Strahlung ist, die zu der Gruppe gehört, die mindestens Folgendes umfasst:
- eine UV-C-Lampe mit Röhre (12), gepulst oder mit kontinuierlicher Welle;
- eine UV-C-Laserlampe;
- mindestens eine UV-C-Leuchtdiode (14), die über einen statischen Konverter mit dem Steuer- und Überwachungssystem (22) des Fahrzeugs verbunden ist.

5. Fahrzeug (10) nach Anspruch 4, wobei eine entsprechende Beleuchtungsvorrichtung mindestens eine UV-C-Leuchtdiode (14) aufweist, die über einen statischen Wandler, der geeignet ist, um mit mindestens einer Leuchtdiode kombiniert zu werden, die konfiguriert ist, um weißes Licht bereitzustellen, mit dem Steuer- und Überwachungssystem (22) des Fahrzeugs verbunden ist.

6. Fahrzeug (10) nach Anspruch 4 oder 5, wobei jede Beleuchtungsvorrichtung geeignet ist, um in eine Beleuchtungsausrüstung integriert zu sein, die ein Glas oder ein transparentes Polymer zum Streuen der von der Beleuchtungsvorrichtung erzeugten Strahlung in das Innere des Fahrzeugs umfasst.

7. Fahrzeug (10) nach einem der Ansprüche 1 bis 6, wobei die Aktivierung mindestens einer der Beleuchtungsvorrichtungen von der Abwesenheit menschlicher Anwesenheit abhängig ist, was von mindestens einem in der Nähe der Beleuchtungsvorrichtung installierten Anwesenheitssensor (32) erfasst wird.

8. Fahrzeug (10) nach einem der vorherigen Ansprüche, ferner umfassend mindestens eine Oberfläche (40, 46), die konfiguriert ist, um die Beleuchtung mit desinfizierender Wellenlänge des Desinfektionssystems zu reflektieren.

9. Fahrzeug (10) nach einem der vorherigen Ansprüche, ferner umfassend eine Rückkopplungsschleife, die mit dem System (22) zur Steuerung und Überwachung des Fahrzeugs verbunden ist und konfiguriert ist, um die Aktivierung der Beleuchtung mit desinfizierender Wellenlänge mittels eines Sensors zu optimieren, der zu der Gruppe gehört, die mindestens Folgendes umfasst:
- einen aktiven Dosimetriesensor, der geeignet ist, um die empfangene Energie, die mit der Strahlung der Beleuchtung mit desinfizierender Wellenlänge assoziiert ist, zu quantifizieren, wobei der aktive Sensor an einer vorbestimmten Stelle innerhalb des Fahrzeugs installiert ist, wobei der Sensor geeignet ist, um die Strahlung der Beleuchtung mit desinfizierender Wellenlänge zu empfangen;
- einen aktiven Dosimetriesensor (34), der geeignet ist, um die empfangene Energie zu quantifizieren, die mit der Strahlung der Beleuchtung mit desinfizierender Wellenlänge assoziiert ist, wobei der aktive Sensor an der Beleuchtung mit desinfizierender Wellenlänge installiert ist und mit einer reflektierenden Oberfläche kombiniert ist, deren Lage innerhalb des Schienenfahrzeugs konfiguriert ist, um die empfangene Strahlung in Richtung des aktiven Sensors, der an der Beleuchtung mit desinfizierender Wellenlänge installiert ist, zu reflektieren;
- einen passiven Sensor, der geeignet ist, um physikalisch auf die Strahlung der Beleuchtung mit desinfizierender Wellenlänge zu reagieren, wobei die physikalische Reaktion des passiven Sensors repräsentativ für eine Strahlung der Beleuchtung mit desinfizierender Wellenlänge ist, die über einem vorbestimmten Schwellenwert ist.

10. Fahrzeug (10) nach einem der vorherigen Ansprüche, wobei das mindestens eine Element des Schienenfahrzeugs, das geeignet ist, um durch die Beleuchtung mit desinfizierender Wellenlänge beleuchtet zu werden, mit einer Beschichtung bedeckt ist, die konfiguriert ist, um es vor einer beschleunigten Alterung durch die Strahlung der Beleuchtung mit desinfizierender Wellenlänge zu schützen.

## Claims

1. A railway vehicle (10), comprising a disinfection system, the disinfection system comprising lighting at a disinfecting wavelength on board said vehicle (10), the lighting comprising at least one lighting device (12, 14) at a disinfecting wavelength, the lighting being configured to automatically disinfect the interior of said vehicle, the vehicle being **characterised in that** said lighting is automatically controlled centrally by a TCMS control and monitoring system (22) of said vehicle (10).

2. The vehicle (10) of claim 1, wherein the disinfectant wavelength lighting comprises a plurality of disinfectant wavelength lighting devices (12, 14) distributed within said vehicle, parallel to the ceiling of said vehicle, in at least one of the following spaces of said vehicle:
- a passenger compartment (18),
- a toilet area (16),
- a driver's cab (20),
- a freight transport area,
- a catering area,
- a passenger entry/exit platform on board said vehicle

3. A vehicle (10) according to claim 2, wherein the control and monitoring system (22) of said vehicle is configured to activate each lighting device (12, 14) according to a predetermined frequency associated with the type of space (16, 18, 20) in which said lighting device is installed.

4. A vehicle (10) according to any one of claims 1 to 3, wherein each lighting device is a UV-C ultraviolet radiation lighting belonging to the group comprising at least:
- a UV-C tube lamp (12), pulsed or with continuous wave;
- a UV-C laser lamp;
- at least one UV-C light-emitting diode (14) connected to the control and monitoring system (22) of said vehicle via a static converter.

5. A vehicle (10) according to claim 4, wherein a corresponding lighting device has at least one UV-C light emitting diode (14) connected to the control and monitoring system (22) of said vehicle via a static converter is adapted to be combined with at least one light emitting diode configured to provide white light.

6. A vehicle (10) as claimed in claim 4 or 5, in which each lighting device is suitable for being integrated within lighting equipment comprising a transparent glass or polymer for diffusing the radiation produced by said lighting device towards the interior of said vehicle.

7. A vehicle (10) according to any one of claims 1 to 6, in which the activation of at least one of the said lighting devices is dependent on the absence of human presence detected by at least one presence detector (32) installed in the vicinity of the said lighting device.

8. A vehicle (10) according to any one of the preceding claims further comprising at least one surface (40, 46) configured to reflect the disinfecting wavelength lighting of said disinfection system.

9. A vehicle (10) according to any one of the preceding claims, further comprising a feedback loop, connected to said system (22) for controlling and monitoring said vehicle, and configured to optimise the activation of said disinfectant wavelength lighting by means of a sensor belonging to the group comprising at least:
- an active dosimetry sensor capable of quantifying the received energy associated with the radiation from said disinfectant wavelength lighting, said active sensor being installed in a predetermined location within said vehicle, wherein said sensor is adapted to receive said radiation from said disinfectant wavelength lighting;
- an active dosimetry sensor (34) suitable for quantifying the energy received associated with said radiation from said disinfectant wavelength lighting, said active sensor being installed at the disinfectant wavelength lighting and combined with a reflective surface whose location within said rail vehicle is configured to cause it to reflect the radiation received in the direction of said active sensor installed at the disinfectant wavelength lighting;
- a passive sensor capable of physically responding to radiation from said disinfectant wavelength illumination, the physical response of said passive sensor being representative of radiation from said disinfectant wavelength illumination in excess of a predetermined threshold.

10. A vehicle (10) according to any one of the preceding claims, in which at least one element of the said rail vehicle suitable for being illuminated by the said lighting at a disinfecting wavelength is covered with a coating configured to protect it from ageing accelerated by the said radiation from the said lighting at a disinfecting wavelength.
